# FASCICULE DE BREVET EUROPEEN

(11) **EP 0 981 356 B1**
(45) Date de publication et mention de la délivrance du brevet: **19.12.2001**
(21) Numéro de dépôt: 98924419.9
(22) Date de dépôt: 12.05.1998
(51) Int. Cl.: A61K 35/78, A61K 7/48, A23L 1/221

(54) **COMPOSITION A BASE D'EXTRAITS VEGETAUX ET D'HUILES ESSENTIELLES, UTILISABLE EN THERAPEUTIQUE, EN COSMETIQUE ET EN DIETETIQUE**
ZUSAMMENSETZUNG AUF DER BASIS VON PFLANZENEXTRAKTEN UND ÄTHERISCHEN ÖLEN, VERWENDBAR IN THERAPEUTIKA, KOSMETIKA UND DIÄTNAHRUNG
COMPOSITION BASED ON PLANT AND ESSENTIAL OIL EXTRACTS, USABLE IN THERAPY, COSMETICS AND DIETETICS

(30) Priorité: 12.05.1997 FR 9705767
(43) Date de publication de la demande: 01.03.2000
(73) Titulaire: Vernin, Jacques, 77000 Melun (FR)
(72) Inventeur: Vernin, Jacques, 77000 Melun (FR)
(74) Mandataire: L'Helgoualch, Jean
(86) Numéro de dépôt international: FR9800943
(87) Numéro de publication internationale: WO9851320

(56) Documents cités:
- WO-A-97/01347
- FR-A- 2 572 935
- PATENT ABSTRACTS OF JAPAN vol. 095, no. 005, 30 juin 1995 & JP 07 039312 A (EZAKI GLICO CO LTD), 10 février 1995
- DATABASE WPI Section Ch, Week 8450 Derwent Publications Ltd., London, GB; Class A96, AN 84-309691 XP002052969 & JP 59 193 818 A (NITTO ELECTRIC IND CO) , 2 novembre 1984

## Description

La présente invention concerne une nouvelle composition utilisable dans les domaines pharmaceutique, diététique et cosmétique, et plus particulièrement une nouvelle composition associant des extraits végétaux titrés et des huiles essentielles procurant des effets potentialisés.

Depuis de nombreuses années, on utilise diverses substances naturelles en raison de leurs propriétés pharmacologiques et cosmétologiques. Les plantes sont parfois utilisées directement, simplement séchées et conditionnées en vrac après récolte, ou coupées à différents degrés, ou encore broyées, pour réaliser des infusions, ou pulvérisées à température ordinaire ou à froid. Dans tous les cas, la conservation des plantes sèches, en vrac ou traitées comme indiqué ci-dessus, ne peut être assurée que pendant une période de temps relativement courte, car une conservation pendant une période de quelques mois, voire quelques semaines, entraîne une perte de leurs propriétés qui sont souvent liées à des composants très volatils ou sensibles à l'oxydation.

La pulvérisation fine d'une plante augmente la surface de contact du contenu des cellules végétales avec l'air ambiant et favorise leur altération, au point qu'il est souvent nécessaire de conserver les poudres sous vide ou sous atmosphère de gaz inerte pour préserver leurs propriétés. De plus, par exemple dans le cas du broyage pour la préparation de poudres, il est souvent nécessaire d'utiliser des broyeurs refroidis à l'azote liquide pour éviter un échauffement qui peut provoquer la perte des substances volatiles.

Parmi les différentes formes de présentation connues, on utilise souvent des extraits secs, ainsi que des huiles essentielles, également dénommées essences végétales de plantes.

Les extraits secs sont généralement obtenus par extraction de la plante sèche avec de l'eau, seule ou additionnée d'éthanol en quantités variables. Les jus d'extraction sont concentrés, puis séchés et additionnés d'excipients destinés à faciliter le séchage et la conservation. Suivant les techniques utilisées, on peut obtenir entre 0,15 g et 1 g environ d'extrait sec pour 2 g de plante sèche.

Les extraits secs préparés par les techniques classiques sont généralement hygroscopiques et leur conservation est difficile.

Les extraits végétaux peuvent aussi être incorporés comme adjuvants dans des compositions, comme décrit par exemple dans le brevet FR-A-2.688.137 relatif à des compositions cosmétiques hydratantes.

Les plantes aromatiques contiennent en moyenne 2 % en poids d'une substance odorante, appelée aussi "essence", qui est une sécrétion naturelle de l'organisme végétal. Une fois extraite du végétal, généralement par distillation à la vapeur d'eau, elle est dénommée "huile essentielle".

En raison de leur composition chimique, la plupart des huiles essentielles ont des propriétés antiseptiques et bactéricides. Selon les espèces botaniques, elles peuvent avoir des propriétés spécifiques qui ont fait l'objet de très nombreuses études depuis des années, et qui ont permis leur utilisation en usage externe comme en usage interne, en cosmétique et en thérapeutique. Les huiles essentielles sont plus particulièrement utilisées en aromathérapie pour le traitement de divers états infectieux, soit comme agent thérapeutique principal, soit comme adjuvant. Par exemple, le brevet FR-A-2.670.386 décrit une composition cosmétique contenant une huile essentielle de basilic destinée à servir de répulsif contre certains insectes.

Cependant, d'une part, les extraits de plantes disponibles dans le commerce présentent généralement une stabilité insuffisante, qui diminue encore leur efficacité, et, d'autre part, on sait que les huiles essentielles sont extrêmement sensibles à l'oxydation, et leur conservation doit donc se faire à l'abri de l'air. De plus, les huiles essentielles doivent être utilisées avec précaution, et on considère qu'il faut éviter de les administrer isolément, mais au contraire qu'il faut les mélanger avec divers excipients en raison des sensations désagréables qu'elles provoquent au contact des muqueuses de la bouche.

Il en résulte que l'efficacité des traitements est fortement limitée, et, même dans le cas de gélules, sachant qu'il faut souvent administrer plus de 10 g de plante par jour pour obtenir un résultat favorable, il faudrait que l'utilisateur absorbe plus de 50 gélules par jour pour pouvoir espérer parvenir à un traitement efficace.

Il en est de même pour les autres formes classiques, telles que infusions, décoctions ou teintures. Ainsi, en utilisant les techniques classiques, on ne peut espérer obtenir des résultats utiles qu'en absorbant chaque jour des quantités au moins égales à 6 infusions ou 60 g de teinture au 1/5 ou de teinture mère homéopathique au 1/10 de plante fraîche, contenant de l'alcool à un titre élevé, de l'ordre de 60 volumes. Il est bien entendu impossible d'envisager d'absorber de telles quantités. De même, les huiles essentielles formulées par les techniques classiques ne peuvent être utilisées que diluées dans des solutions alcooliques fortement titrées.

Le brevet FR-A-2 572 935 décrit une composition à usage alimentaire comprenant un substrat constitué par une poudre végétale, contenant une huile essentielle provenant du même végétal. L'huile essentielle doit être chauffée pour être incorporée dans le substrat, et il en résulte une volatilisation de la plus grande partie de l'huile, ainsi qu'une certaine instabilité de la composition.

Il existe donc un besoin de compositions efficaces et stables, utilisables en quantités réduites, et permettant l'administration simple des principes actifs essentiels des plantes.

La présente invention a pour objet de nouvelles compositions utilisables en thérapeutique, en diététique et en cosmétique, comprenant des extraits végétaux titrés en principes actifs, et des huiles essentielles, en mélange, si nécessaire, avec des excipients pharmaceutiquement ou cosmétiquement acceptables, et présentant une excellente stabilité dans le temps.

Les nouvelles compositions suivant la présente invention se présentent de préférence sous forme de gélules ou de capsules administrables par voie orale. Elles peuvent aussi être présentées sous une forme administrable par voie topique, par exemple un gel.

Suivant une forme préférentielle de réalisation de l'invention, les huiles essentielles utilisées dans les compositions, sont sous forme de microcapsules.

Les microcapsules contiennent une huile essentielle et sont associées de préférence à un tampon. Le tampon utilisé dans l'invention est mélangé aux microcapsules et est généralement constitué par un bicarbonate ou un hydrogéno carbonate, un tampon phosphate, ou encore l'acide éthylène diamine tétracétique. De préférence on utilise du bicarbonate de sodium. Le tampon facilite la tolérance gastrique et permet en outre d'éviter de devoir procéder à un enrobage entérique des gélules contenant la composition de l'invention. Un mélange de microcapsules contenant des huiles essentielles différentes peut être utilisé conformément à la présente invention.

La taille dés microcapsules utilisées dans la présente invention est généralement comprise entre 25 µm et 0,5 mm, et de préférence entre 50 µm et 300 µm. Les polymères utilisés pour former la paroi des microcapsules peuvent être choisis parmi ceux couramment utilisés dans les techniques pharmaceutiques et cosmétiques. Suivant l'invention, on utilise de préférence de la gélatine, une gomme arabique ou une gomme adragante, isolément ou en mélange.

Conformément à l'invention, on peut préparer par exemple des gélules ou capsules contenant entre 0,05 g et 0,50 g d'extrait sec titré et entre 0,05 g et 0,50 g de microcapsules d'huile essentielle, par gélule ou capsule. Ces quantités ne sont pas limitatives et peuvent varier en fonction de la taille des gélules ou des capsules.

Les compositions de l'invention permettent aussi d'associer avantageusement des extraits secs titrés en principes actifs de plusieurs plantes avec une ou plusieurs essences essentielles microencapsulées et tamponnées d'autres plantes.

Dans le cas de l'application en dermatologie ou en cosmétologie, les compositions de l'invention peuvent être aisément adaptées à un usage externe en incorporant, à un excipient approprié, des extraits végétaux liquides ou secs, titrés ou non, et des microcapsules d'huiles essentielles. Ainsi, il est possible de préparer des compositions conformes à la présente invention sous forme de gel pour application topique. Un excipient approprié peut être par exemple un gel tel qu'un gel de Carbopol.

Les compositions conformes à la présente invention possèdent divers avantages par rapport aux formes classiques de présentation de substances naturelles. En particulier, elles sont totalement assimilables et présentent une biodisponibilité améliorée, une excellente stabilité dans le temps sans qu'il soit nécessaire de les conserver dans des conditions très rigoureuses de température et de degré d'hygrométrie, et elles procurent une potentialisation des effets des principes actifs naturels utilisés. De plus, elles préservent l'intégrité des huiles essentielles vis-à-vis de leur volatilisation et de leur oxydation, et la microencapsulation prévient les risques de réaction entre les constituants des huiles essentielles associées, le cas échéant. Enfin, la tolérance, notamment la tolérance gastrique, est améliorée, même en cas de composition contenant des mélanges de microcapsules, tandis que l'administration d'un mélange d'huiles essentielles entraîne généralement un effet irritant pour les muqueuses.

Plus particulièrement, les compositions suivant la présente invention procurent une action immédiate due aux microcapsules d'huiles essentielles, complétée par une action prolongée provenant des extraits végétaux.

Ainsi, les essais effectués avec diverses compositions conformes à la présente invention ont montré qu'une gélule contenant des extraits végétaux secs titrés en principe actif et des microcapsules d'huiles essentielles, ont une activité équivalente à celle de 10 à 30 gélules classiques de plantes sèches.

Les exemples suivants illustrent l'invention plus en détail sans en limiter la portée. Dans ces exemples, les parties sont données en poids, sauf indication contraire.

### Exemple 1

On prépare par une technique connue (extraction hydroalcoolique, concentration à 35°, puis séchage sous vide), un extrait sec de romarin concentré à 1:8 et titré, c'est-à-dire 0,15 g d'extrait sec pour 1,2 g de plante.

D'autre part, par une technique de microencapsulation par coacervation, on prépare des microcapsules (teneur en gélatine : 20%) contenant une essence tamponnée de romarin (0,15 g d'essence pour 3,35 g de plante). Le tampon utilisé est le bicarbonate de sodium.

Après mélange des microcapsules, du tampon et de la poudre d'extrait sec titré de romarin, dans un mélangeur cubique Frogerais, on obtient une composition que l'on incorpore dans des gélules (gélule n° 1) en utilisant un appareil à remplissage par arasement.

La composition de la gélule est donc :

| | |
|---|---|
| Extrait sec titré de romarin (1:8) | 0,15 g |
| Microcapsules d'essence tamponnée de romarin | 0,15 g |

Une gélule de la composition ainsi préparée correspond à 4,55 g de plante sèche, soit environ 22 à 23 gélules de poudre de plante suivant une technique classique.

Cette composition présente des propriétés de tonique digestif favorisant la digestion permettant son utilisation comme complément diététique.

### Exemple 2

En utilisant la même technique de préparation que dans l'Exemple 1, on prépare une poudre d'extrait sec titré de bardane (1:5) en mélange avec une poudre d'extrait sec titré de houblon (1:5).

D'autre part, on prépare des essences microencapsulées tamponnées de thym et des essences microencapsulées tamponnées de menthe.

On mélange l'ensemble et on obtient ainsi une gélule de 400 mg ayant la composition suivante :

| | |
|---|---|
| Extrait sec titré de bardane (1:5) | 0,10 g |
| Extrait sec titré de houblon (1:5) | 0,10 g |
| Microcapsules d'essence tamponnée de thym | 0,10 g |
| Microcapsules d'essence tamponnée de menthe | 0,10 g |

Une gélule de la composition ainsi préparée correspond à 2,8 g de plante sèche, soit environ 10 gélules de poudre de plante suivant une technique classique.

Cette composition présente des propriétés de tonique hépatique et antiseptique cutané, permettant son utilisation en thérapeutique et en diététique pour le traitement des manifestations cutanées.

### Exemple 3

On prépare une composition sous forme de gel dermique pour utilisation dermatologique par application topique, ayant la composition suivante :

| | |
|---|---|
| extrait hydroalcoolique d'harpagophytum | 40 g |
| extrait hydroalcoolique de baies de genièvre | 10 g |
| essence microencapsulée tamponnée d'eucalyptus citriodora | 2 g |
| excipient spécial d'absorption instantanée | 50 g |

On obtient ainsi un gel dermique ayant des propriétés anti-inflammatoires.

## Revendications

1. Composition utilisable en cosmétique, en diététique et en thérapeutique, **caractérisée en ce qu'**elle contient en combinaison
des extraits végétaux titrés en principes actifs
des huiles essentielles microencapsulées,
un tampon associé aux microcapsules d'huiles essentielles,
en mélange, si nécessaire, avec des excipients pharmaceutiquement ou cosmétiquement acceptables.

2. Composition selon la revendication 1, **caractérisée en ce qu'**elle se présente sous forme de gélule ou de capsule.

3. Composition selon la revendication 1, **caractérisée en ce que** la taille des microcapsules est comprise entre 25 µm et 0,5 mm.

4. Composition selon la revendication 1, **caractérisée en ce que** le tampon est le bicarbonate de sodium.

5. Composition selon l'une quelconque des revendications précédentes, **caractérisée en ce qu'**elle contient en outre un excipient adapté à un usage externe de la composition.

6. Composition selon l'une quelconque des revendications 1 à 4, **caractérisée en ce qu'**elle contient entre 0,05 g et 0,50 g d'extrait sec titré et entre 0,05 g et 0,50 g de microcapsules d'huile essentielle, par gélule ou capsule.

## Claims

1. Composition which can be used in the cosmetic, dietetic and therapeutic fields, **characterized in that** it contains in combination
- plant extracts with defined active ingredient titers
- individually microencapsulated essential oils,
- a buffer associated with the microcapsules of essential oils,
in the form of a mixture, if necessary, with pharmaceutically or cosmetically acceptable excipients.

2. Composition according to claim 1, **characterized in that** it is provided in the form of a gelatin capsule or a capsule.

3. Composition according to claim 1, **characterized in that** the size of the microcapsules is between 25 µm and 0.5 mm.

4. Composition according to claim 1, **characterized in that** the buffer is sodium bicarbonate.

5. Composition according to any one of the preceding claims, **characterized in that** it contains, in addition, an excipient suitable for an external use of the composition.

6. Composition according to any one of claims 1 to 4, **characterized in that** it contains between 0.05 g and 0.50 g of dry extract of defined titer and between 0.05 g and 0.50 g of microcapsules of essential oil, per gelatin capsule or capsule.

## Patentansprüche

1. In Kosmetik-, Diät- und Therapiemitteln einsetzbare Zusammensetzung, die **dadurch gekennzeichnet ist, dass** sie eine Kombination von:
Pflanzenextrakten mit bestimmtem Wirkstoffgehalt,
essentiellen Ölen in Mikrokapseln,
einem den Mikrokapseln mit essentiellen Ölen zugeordneten Puffer,
falls erforderlich im Gemisch mit pharmazeutisch oder kosmetisch annehmbaren Exzipienten, umfasst.

2. Zusammensetzung nach Anspruch 1, **dadurch gekennzeichnet, dass** sie in Gelatinekapsel- oder Kapselform vorliegt.

3. Zusammensetzung nach Anspruch 1, **dadurch gekennzeichnet, dass** die Größe der Mikrokapseln zwischen 25 µm und 0,5 mm liegt.

4. Zusammensetzung nach Anspruch 1, **dadurch gekennzeichnet, dass** der Puffer Natriumbicarbonat ist.

5. Zusammensetzung nach einem der vorangegangenen Ansprüche, **dadurch gekennzeichnet, dass** sie außerdem einen für eine äußerliche Anwendung der Zusammensetzung geeigneten Exzipienten enthält.

6. Zusammensetzung nach einem der Ansprüche 1 bis 4, **dadurch gekennzeichnet, dass** sie zwischen 0,05 g und 0,50 g trockenen Extrakt mit bestimmtem Gehalt und zwischen 0,05 g und 0,50 g der Mikrokapseln mit essentiellen Ölen pro Gelatinekapsel oder Kapsel enthält.
